# EUROPEAN PATENT APPLICATION

(11) **EP 1 829 964 A1**
(43) Date of publication of application: **05.09.2007**
(21) Application number: 05809430.1
(22) Date of filing: 24.11.2005
(51) Int. Cl.: C12N 15/09, C12Q 1/68

(54) **METHOD OF EXAMINING GENE SEQUENCE**

(30) Priority: 08.12.2004 JP 2004354915
(71) Applicant: Yamamoto, Takeshi, Yokohama-shi Kanagawa 244-0813 (JP)
(72) Inventor: Yamamoto, Takeshi, Yokohama-shi Kanagawa 244-0813 (JP)
(74) Representative: Krauss, Jan
(86) International application number: PCT/JP2005/021554
(87) International publication number: WO 2006/061994

(57) **Abstract**

The present invention provides a method for detecting quantitatively a specific nucleic acid sequence and for detecting gene polymorphism or mutation by homogenous system simply, promptly, accurately, and inexpensively, and an oligonucleotide probe used therefor. The nucleic acid having a specific sequence is quantitatively detected and gene polymorphism or mutation is detected in such a way that a probe DNA containing one or two labeling materials is hybridized to the target nucleic acid, and decomposed from the 5'- or the 3'-terminal by exonuclease action to release one of the labeling materials, the signal emitted by which is then detected.

## Description

### Technical Field

The present invention relates primarily to a method of gene analysis in the fields of clinical medicine, forensic medicine, pharmacological chemistry, biochemistry, food chemistry, agricultural chemistry, or the like, which is used for analysis of mutations or polymorphisms in genomic genes and genes of viruses, bacterium or the like.

### Background Art

Gene polymorphism (hereinafter referred to as "polymorphism") denotes diversification of genome which is found to exist at a frequency of 1% or more in all individuals, and includes, in addition to single nucleotide polymorphism (SNP), deletion or insertion of from one to several tens bases, difference in number of repeated sequence comprising from several bases (microsatellite) to several tens bases.

Analysis of mutation or polymorphism in gene sequence is important in diversified fields including clinical medicine, forensic medicine, pharmacological chemistry, biochemistry, food chemistry, agricultural chemistry, or the like. It has been shown that mutation in human gene (normally occurring at a frequency of less than 1%) is deeply associated with hereditary disorders, cancers, or the like, and that polymorphism (normally occurring at a frequency of 1% or more) is association with risk of disorders, drug sensitivity, drug metabolic rate, or the like. Further, those in viruses and bacteria genes are association with drug tolerance and pathogenicity. Furthermore, they are also used for identification of an individual, breed identification of agricultural products, or the like.

At present, in order to analyze mutation or polymorphism in a gene sequence with best accuracy, the Sanger method is primarily used to determine the nucleotide sequence. The procedure of nucleotide sequence determination has recently automated in part, but is still cumbersome and needs longer time for analysis, and expensive apparatus and reagents.

As a simple, fast and inexpensive method for polymorphism analysis, there have been developed other methods than nucleotide sequence determination by the Sanger method. Particularly, a method based on hybridization is widely and commonly used. The hybridization-based method is a method wherein a probe having normally a length of about 15 to 25 bases is fixed on a solid phase such as a microplate, a membrane, a bead or a glass slide, and hybridized with a target nucleic acid to form a heteroduplex, which is, during or after the hybridization, washed at an appropriately adjusted condition of pH, salt concentration and temperature to dissociate or remove selectively only the heteroduplex having a mismatched base pair, because the heteroduplex having the mismatched base pair has a different melting temperature (hereinafter referred to as Tm) from the heteroduplex having no mismatched base pair to give a difference (hereinafter referred to as ΔTm). This is based on so-called B/F separation method. Now, a probe, which has a longer sequence, brings an increased Tm to decrease the share of ΔTm in the whole Tm. This decreased share of ΔTm makes it difficult to separate target nucleic acids between with and without a mismatch through an adjusted condition for washing. Meanwhile, a probe, which has a shorter sequence, not only reduces specificity of hybridization but also reduces the whole Tm to decrease its own binding affinity to a nucleic acid, thereby to fail in specific and effective hybridization. The probe having low affinity is often difficult to hybridize even under a mild condition so that a match target nucleic acid and a mismatch one are not discriminated and both removed.

As the other method, there is a method called mini-sequencing. This method is a method wherein a DNA to be examined is used as a template to incorporate nucleotides of one to several bases by primer extension, and then the polymorphism is analyzed by identification of the nucleotide incorporated according to the sequence of the template DNA. The mini-sequencing method often uses a DNA amplified by PCR as a template, and thus needs various complicated treatments before the primer extension, such as removal of the remaining PCR primer and nucleotide, and purification of the PCR product to exchange the reaction solution. Further, the method often uses mass spectrometry to identify the incorporated nucleotide in type as up-to-date method, and thus needs a very expensive apparatus. The mini-sequencing method has problems associated with economy and promptness.
Patent Document 1: Japanese Patent Application Laid-Open No. 6-327499
Patent Document 2: International Patent Publication WO2002/077282
Patent Document 3: International Patent Publication WO2003/035864
Patent Document4: International Patent Publication WO01/23583
Non-Patent Document 1: Bio Techniques, 13, 888~892, 1992
Non-Patent Document 2: Proc. Natl. Acad. Sci. USA, 58 (2), 719~726 (1967)
Non-Patent Document 3: Biochemistry, 37, 10156~10163 (1998)
Non-Patent Document 4: Tetrahedron Lett., 22, 1859 (1981)
Non-Patent Document 5: Biochemistry, 38, 3468~3477 (1999)
Non-Patent Document 6: Nucleic Acids Res. 28, e63 (2000)
Non-Patent Document 7: Clinical pathology 50, 528~532 (2002)
Non-Patent Document 8: Proc. Natl. Acad. Sci USA 92 (10), 4641~4645 (1995)

### Disclosure of the Invention

### Problems to be Solved by the Invention

The present invention relates to a method for examining gene sequences using an exonuclease, and provides a simple, rapid, and highly accurate homogeneous detection method, that is, a technique remarkably improved in sensitivity and specificity for detecting a difference in gene sequence based on a difference in Tm caused by a mismatch between a target nucleic acid and a probe, though the detecting has been conventionally difficult. The present invention also provides an economically excellent detection technology, because it uses nothing but commercially available and inexpensive materials and reagents.

### Means for Solving the Problems

The cycling assay, which uses λ exonuclease (Non-Patent Document 1) or exonuclease III (Patent Document 1) specifically digesting a double-stranded DNA, hybridizes an oligonucleotide probe labeled with a radioactive material to the complementary sequence to form a double-stranded DNA, which is then digested with an exonuclease to give a shortened probe. The shortened probe is dissociated from the target nucleic acid, then newly given probe is hybridized, and digested. Such a process is repeated to establish a cycling reaction. The digested products from the probe are detected by autoradiography or scintillation counter to determine whether or how much the target nucleic acid is found.

. Further, the homogeneous detection system of exonuclease cycling assay without using a radioactive material is reported (Patent Documents 2 and 3). The detection system uses an oligonucleotide probe labeled with a fluorescent dye and a quenching material (quencher). The undigested probe emits no or faint fluorescence because fluorescence energy emitted from the fluorescent dye is absorbed by the quencher, while the probe digested by an exonuclease releases the fluorescent dye, which then comes apart from the quencher at a distance enough for the fluorescence energy to exempt from the absorption. As a result, the fluorescent dye can emit an inherent wavelength of fluorescence, which is detected to determine the amount of the digested probe. Such a method wherein a fluorescent dye and a quencher that can effectively absorb the fluorescence energy of the dye are used as the labeling materials is known generally as fluorescence resonance energy transfer (FRET) (Non-Patent Document 2).

As mentioned above, although it is known that a specific target sequence can de detected quantitatively by exonuclease cycling assay, it has not been shown that gene polymorphism can be detected by exonuclease cycling assay. The present inventor made an intensive study and have found that the homogeneous detection system of exonuclease cycling assay can identify simply, promptly, inexpensively, and accurately a difference of as few as one or several bases in base sequence on a target gene.

In order to improve the specificity of hybridization of a probe, the probe preferably has a sufficiently large base length. However, to sensitively and specifically determine the presence or the absence of a mismatch between the probe and a target nucleic acid (hereinafter, "mismatch" includes a non-complementary base pair or loop structure, unless otherwise specifically shown) is generally difficult by detecting whether the hybridization occurs or not, wherein the probe with the length of 25 or more bases is used. This is because a longer probe used gives too little a difference in Tm between with and without a mismatch (ΔTm) to allow distinction through an adjustment of washing condition for B/F separation. The present invention based on hybridization can use a sufficiently long probe such as a probe having 25 or more bases, and allows hybridization to a target nucleic acid sequence at a very high affinity even if the sequence has polymorphism. Further, the present invention can identify polymorphism more sensitively and specifically than a conventional hybridization method using a longer probe, because the present invention can use an increased share of ΔTm in the whole Tm given by a mismatch when a probe is digested by an exonuclease to have a shortened length of about 5-15 bases. But this is not always true since polymorphism can be condition-dependently detected.

Moreover, the present invention is simple to operate and does not require a washing step for removing a probe having a mismatch with a target nucleic acid, or a target nucleic acid having a mismatch with a probe, that is, so-called B/F separation, as required by conventional hybridization method. The present invention can be applied immediately to exonuclease cycling assay without purifying products amplified by gene amplification method, and does not need denaturation of a double-stranded DNA to be used as a test specimen, and so is extremely excellent in promptness and simplicity. All reagents necessary for the present invention are commercially available at inexpensive price, and instruments and apparatuses needed are those used most commonly in gene analysis, and therefore, the present invention is extremely excellent in economy and versatility.

Namely, the present invention comprises:
1. A method of homogenous detection system for discriminating a target nucleic acid having a specific sequence from the other nucleic acid which has the sequence of the target nucleic acid comprising at least one or more bases substitution, insertion or deletion, thereby to detect quantitatively, the method comprises the steps of:
   I) hybridizing an oligonucleotide probe containing a labeling material to the target DNA;
   II) digesting the hybridized oligonucleotide probe by a double-stranded DNA-specific 5'→3' or 3'→5' exonuclease; and
   III) detecting a signal emitted by at least one labeling material released from the digested oligonucleotide probe.
2. The method according to above-mentioned 1, wherein the target nucleic acid, which has a specific site to form at least one or more non-complementary base pairs with the probe or has the sequence of the target nucleic acid with one or more bases substituted, inserted, or deleted to form a loop structure, does not hybridize with the probe or hybridizes, but leaves the probe before digestion by a 5'→3' exonuclease or a 3'→5' exonuclease, so that the probe is exempted from digestion to release no origonucleotide containing the labeling material, resulting in no significant increase of the signal.
3. The method according to above-mentioned 1 or 2, wherein the exonuclease is a 5'→3' exonuclease, and the oligonucleotide probe is designed to form the at least one or more non-complementary base pairs or the loop structure with regard to the target nucleic acid so that the base pairs or the loop structure may be positioned between the 3'-terminal of the probe and a site labeled with the labeling material.
4. The method according to above-mentioned 1 or 2, wherein the exonuclease is a 3'→5' exonuclease, and the oligonucleotide probe is designed to form the at least one or more non-complementary base pairs or the loop structure with regard to the target nucleic acid so that the base pairs or the loop structure may be positioned between the 5'-terminal of the probe and a site labeled with the labeling material.
5. An oligonucleotide probe for use in the method according to above-mentioned 3, wherein the labeling material is a fluorescent dye, toward the 3'-side from which the probe is labeled with a quenching material which has an effect to attenuate efficiently fluorescence emitted by the fluorescent dye.
6. An oligonucleotide probe for use in the method according to above-mentioned 3, wherein the labeling material is a quenching material, toward the 3'-side from which the probe is labeled with a fluorescent dye the fluorescence of which can be attenuated efficiently by the quenching material.
7. An oligonucleotide probe for use in the method according to above-mentioned 4, wherein the labeling material is a fluorescent dye, toward the 5' -side from which the probe is labeled with a quenching material which has an effect to attenuate efficiently the fluorescence emitted by the fluorescent dye.
8. An oligonucleotide probe for use in the method according to above-mentioned 4, wherein the labeling material is a quenching material, toward the 5'-side from which the probe is labeled with a fluorescent dye the fluorescence of which can be attenuated efficiently by the quenching material.
9. An oligonucleotide probe for use in the method according to above-mentioned 3 or 4, wherein the labeling material is a fluorescent nucleotide analog which can form a Watson-Crick base pair with any of guanine, adenine, thymine, and cytosine in the target DNA.
10. The oligonucleotide probe according to above-mentioned 9, wherein the fluorescent nucleotide analog is 2-aminopurine.
11. The method according to above-mentioned 3, wherein the 5'→3' exonuclease is λ exonuclease or T7 Gene6 exonuclease.
12. The method according to above-mentioned 4, wherein the 3'→5' exonuclease is exonuclease III.
13. The method according to any one of above-mentioned 1 through 4, wherein the exonuclease is a thermostable enzyme.
14. The method according to above-mentioned 13, wherein the thermostable enzyme is derived from Archaeoglobus fulgidus.
15. The method according to above-mentioned 1 or 2, wherein a cDNA synthesized from RNA by reverse transcription reaction, or a product amplified by polymerase chain reaction (PCR) or reverse transcription-PCR (RT-PCR) is not purified to be used as a test specimen.
16. The method according to any one of above-mentioned 1 to 4 and 11 to 15, comprising using the oligonucleotide probe according to any one of above-mentioned 5 to 10, wherein the oligonucleotide probe has been intentionally introduced with the one or more bases of substitution, insertion or deletion to form a non-complementary base pair or a loop structure with regard to the target nucleic acid, thereby to get the improved ability to discriminate the target nucleic acid from the nucleic acid which has the sequence of the target nucleic acid comprising at least one or more bases of substitution, insertion or deletion.
17. A measurement reagent or a kit for use in the method according to any one of above-mentioned 1 through 16.

### Advantages of the Invention

The present invention allows discrimination of a target nucleic acid having a specific nucleotide sequence from a nucleic acid which has the nucleotide sequence comprising one or more bases substitution, insertion, or deletion, thereby to detect quantitatively. Further, the present invention does not need expensive reagents or materials, and thus is satisfied with a low running cost. The present invention provides a rapid assay using simple operations, because it does not need purification and denaturation of products after PCR reaction and allows real-time measurement of a signal.

### Description of the Preferred Embodiment

The present invention is a method for quantitatively detecting a target nucleic acid sequence and identifying polymorphism or mutation in the target nucleic acid sequence. The present invention utilizes a mechanism wherein an oligonucleotide probe bound with a labeling material (hereinafter referred to as "probe") is specifically hybridized to a target nucleic acid sequence, and only the probe, which has a completely complementary sequence with the sequence around the polymorphism site or the mutation site in the target nucleic acid sequence, is digested by an exonuclease to release the labeling material, thereby to give the signal.

The labeling material according to the present invention is involved in generation or control of a signal, and more specifically, is a material which emits fluorescence, or a material which attenuates fluorescence emitted by the other material. A probe DNA of one molecule includes one or two labeling materials.

When one labeling material is used, a fluorescent nucleotide analog may be used as the labeling material. The fluorescent nucleotide analog, when it is a mononucleotide, emits strong fluorescence, while the analog, when it is contained in a polynucleotide or oligonucleotide, emits attenuated fluorescence and cooperates with the other base to form a Watson-Crick base pair. As such nucleotide analog, 2-aminopurine can be exemplified, though the present invention is not limited thereto. 2-aminopurine is substituted for adenine to form a base pair with thymine, and a nucleic acid containing 2-aminopurine can be a substrate for exonuclease (Non-Patent Document 3). Accordingly, a probe containing 2-aminopurine, which is bound to a target nucleic acid in the exonuclease cycling assay, is digested by exonuclease to release 2-aminopurine, the fluorescence emitted by which is then detected to allow homogeneous detection of a specific nucleic acid sequence.

When two labeling materials are used, a fluorescent dye and a material having a property to effectively attenuate fluorescence thereof (hereinafter referred to as "quencher") may be used as the labeling material. The combination of a fluorescent dye and a quencher for effectively attenuating fluorescence thereof may include any combination as long as it has such a function, and further, is not particularly limited as long as it substantially interfere neither hybridization of a probe DNA and a target nucleic acid, nor digestion by exonuclease. Specifically, a fluorescent dye such as fluorescein, FITC, FAM, TAMRA, Cy3, Cy5, and europium is combined with another fluorescent dye as the quencher which has an absorption wavelength coinciding with or in the vicinity of the fluorescence wavelength and emits a longer wavelength of fluorescence to give what is known generally as fluorescence resonance energy transfer (FRET) (Non-Patent Document 2). Moreover, a non-fluorescent dye which radiates absorption energy as heat in lieu of fluorescence may be used as the quencher. This sort of material is referred to as dark quencher, and many of them are excellent in fluorescence attenuating efficiency. Specifically, Dabcyl, Eclipse, and BHQ (Black Hole Quencher) may be exemplified.

Out of two labeling materials, the one that is released from a probe by exonuclease reaction may be either a fluorescent dye or a quencher. However, in either case, it is necessary that the quencher be arranged so as to attenuate effectively fluorescence of the fluorescent dye on the probe and that either of the fluorescent dye or the quencher which is released from the probe should be detected as an increase in fluorescence intensity.

Single-stranded DNA utilized as a probe is normally synthesized organic-chemically by the amidite method (Non-Patent Document 4). It is known to those skilled in the art that a fluorescent dye and quencher-labeled probe can be easily synthesized using their respective labeling amidite reagents and an automatic synthesis apparatus. Further, DNA synthesized using an amidite reagent subjected to amination can be coupled with a labeling material introduced with an activated ester such as succinimide. Labeled DNAs produced by these methods are available through commercial services.

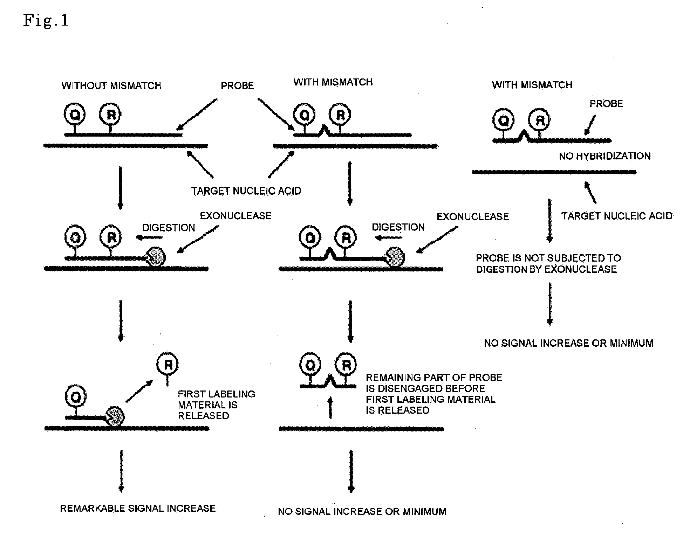

In either case where one or two labeling materials are used, the labeling material (when two labeling materials are used, at least one of them) is released by activity of exonuclease only when the sequence of a specific region in the target nucleic acid is completely complementary to the probe sequence (left part of FIG. 1). The target nucleic acid and the probe give a lower Tm to fail in hybridization when the target nucleic acid sequence has polymorphism or mutation in the specific region to form a one base mismatch or a two or more base mismatch or a loop structure in combination with the probe sequence than when they are completely complementary (right part of FIG. 1) . Alternatively, the probe hybridizes with the target nucleic acid and then digested by an exonuclease to bring a shortened length of probe, give a lowered Tm to fail in the double strand to keep, so that the probe is disengaged from the target nucleic acid before the probe releases the labeling material by the exonuclease, resulting in no or the least increase in fluorescence (center part of FIG. 1) . The method according to the present invention is characterized by utilizing these facts to identify polymorphism or mutation in a target nucleic acid.

When a 5' →3' exonuclease is used for the exonuclease, the probe is designed to have the polymorphism or mutation site of a target nucleic acid positioned between a labeling site for a first labeling material and the 3'-temrinal of the probe (hereinafter, the labeling material released first from the probe by exonuclease reaction is referred to as "first labeling material." When the probe includes one labeling material, the "first labeling material" denotes the labeling material itself). Meanwhile, when a 3'→5' exonuclease is used for the exonuclease, the probe is designed to have the polymorphism or mutation site of a target nucleic acid positioned between a labeling site for a first labeling material and the 5'-temrinal of the probe.

A site of mismatch in the hybrid of the probe and a target nucleic acid, which is located near the terminus of a probe, has less influence on the stability of the hybrid of the probe and the target nucleic acid than a mismatch which is located around the center of the probe. Therefore, the probe, which has a sufficiently long chain length, can hybridize efficiently to the target nucleic acid whether the probe has a mismatch or not. Then, the probe hybridized to the target nucleic acid is digested by the activity of exonuclease to become shorter with the lowered Tm, thereby to make the hybrid unstable. On this occasion, the probe and the target nucleic acid, if they have a mismatch in one or more bases therebetween, have a further lowered Tm to bring a further unstable hybrid, compared with a hybrid without the mismatch. As a result, the probe is disengaged from the target nucleic acid before it releases the first labeling material by exonuclease (FIG. 1).

The labeling site of a first labeling material should be determined taking account of the chain length of a remaining probe shortened by exonuclease when it is disengaged from a target nucleic acid. Particularly, the remaining probe, which has no mismatch with regard to a target nucleic acid, releases the first labeling material by exonuclease and then is disengaged from the target nucleic acid. Meanwhile, the remaining probe, which has a mismatch with regard to the target nucleic acid, is disengaged from the target nucleic acid before it releases the first labeling material by exonuclease or does not hybridize. For this reason, the labeling site of a first labeling material should be determined to prevent the first labeling material from being released when the hybrid has a mismatch.

A probe, which is completely complementary to a target nucleic acid, is disengaged from the target nucleic acid to give a chain length depending on base composition in the remaining probe, reaction temperature, solution pH, and salt concentration. In general, the higher the content of guanine and cytosine, the lower the reaction temperature, the lower the pH and higher the salt concentration, the shorter the chain length when the remaining probe is disengaged from the target nucleic acid. Therefore, the general range of condition for executing the present technology (reaction temperature from 25°C to 45°C, pH from 7.0 to 10.0, salt concentration from 10 mM to 200 mM) gives a chain length of about 5 to 15 bases when a probe DNA is disengaged from a target DNA, although the present invention is not limited thereto. Therefore, the site for labeling the first labeling material on the probe is preferably positioned at a nucleotide which is any one of 6-th to 16-th bases counted from the 3'-terminus for a 5'→3' exonuclease used for the exonuclease, or is any one of 6-th to 16-th bases counted from the 5'-terminus for a 3'→5' exonuclease used for the exonuclease. Reaction conditions should be adjusted so that the probe having a mismatch with regard to the target nucleic acid may have a significantly longer length when it is disengaged from the target nucleic acid than the probe having no mismatch.

However, depending on a nucleotide sequence around a polymorphism site, in some cases, the probe digested by exonuclease does not have a remarkably different length between with and without a mismatch when it is disengaged from the target nucleic acid. Especially in many cases, the probe and the target nucleic acid, which have a high GC content of region and therefore has a comparatively high Tm, have generally a ΔTm which has no great influence even if they have a mismatch in as few as one base. Further, a mismatch consisting of G/G pair and a mismatch consisting of G/T pair have relatively stable hydrogen bonds compared with the other mismatch base pairs (Non-Patent Document 5), and therefore, the mismatched base pairs in a high GC content region are generally difficult to identify through the ΔTm by a conventional method based on hybridization. In this case, such probe can be improved by introducing intentional substitution, insertion or deletion in a part of the probe to cause other mismatches with regard to the target nucleic acid. The site and the manner (such as type of bases to substitute, insert, or delete) for introducing artificial mismatches are not particularly limited as long as they can provide a remaining part of the probe digested by exonuclease with a significant difference in length between with and without a mismatch attributable to polymorphism in the target nucleic acid when the remaining part is disengaged from the target nucleic acid. For example, the G/C base pair in a high GC content region of a probe is partly substituted to form a mismatch consisting of G/G, G/T or G/A base pair, thereby to cause the probe and the target nucleic acid to have a lowered Tm around the polymorphism site, allowing a ΔTm between with and without the mismatch to have a great influence. Further, the probe is introduced with such an artificial mismatch, allowing the probe and the target nucleic acid to form a hybrid only when they are completely matched in a polymorphism site and no hybrid otherwise.

One of advantages of the present invention is that although it is a method for detecting polymorphism or mutation based on hybridization, there is no particular limitation to chain length of a probe used. For example, there are approximately 4.3 billion types of probes for a 16 base length produced from four types of bases by random permutations and combinations. A human genome has approximately 3 billion base pairs, and therefore, a length of 16 or more 16-bases is necessary to produce a highly specific probe for the human genome, and the hybridization method or the PCR method, which requires high specificity, normally uses a probe or a primer having a length of 20 to 30 bases. However, since the conventional method for identifying a difference in base uses B/F separation by washing after hybridization, a probe to use is limited in length. Namely, the longer the probe length, the smaller the share of ΔTm in Tm of full-match, causing difficult discrimination from a mismatch. Therefore, the probe has preferably a short length as far as possible. However, as mentioned previously, the probe has a shortened length in order to increase the share of ΔTm in Tm of full-match, to bring a problem that it reduces specificity and affinity to the target nucleic acid.

According to the present invention, the probe is not particularly limited in length as far as it keeps a minimal chain length, the suitable site in which can be then labeled with a first labeling substance, that is, a signal generating material which can be released by an exonuclease to be specific to the target sequence. Therefore, the present invention can analyze about a living organism having a large genome size by lengthening a probe to keep sufficient specificity and affinity. An ordinary synthesis apparatus can produce an oligomer DNA having a length of 100 or more bases, but a full-length DNA is produced with lower synthesis efficiency as the chain length gets longer, and thus a probe having an excessively long chain is used to be economically disadvantageous. Moreover, the longer chain length needs a longer time for digestion by exonuclease, and the cycling reaction is unlikely to take place effectively. Therefore, the method according to the present invention preferably uses a probe having a length of 18 to 50 bases, but also can use a probe without this range.

The site for labeling a second labeling material in a probe (hereinafter, when two labeling materials are included in a probe, another labeling material for forming a pair with the first labeling material to establish FRET phenomenon is referred to as "second labeling material") is not particularly limited as long as it is not released prior to the first labeling material by exonuclease and can attenuate effectively fluorescence of the first labeling material. For example, there can be used a probe the 3'-terminus of which is labeled with the second labeling material for a 5'→3' exonuclease used for the exonuclease, while the 5'-terminus of which is labeled with the same material for a 3'→5' exonuclease used for the exonuclease.

However, the first labeling material and the second labeling material are apart from each other at an increased distance to weaken the effect of the quencher to attenuate fluorescence, thereby, in some cases, to increase fluorescence signal of the background. In this case, the second labeling material can be labeled within the chain of the probe in order to shorten a distance between the both labeling materials. Namely, the second labeling material can be labeled in the probe chain at a nucleotide between the 3' -terminal and the first labeling material for a 5'→3' exonuclease used for the exonuclease, while at a nucleotide between the 5' -terminal and the first labeling material for a 3'→5' exonuclease used for the exonuclease. This arrangement can prevent the second labeling material from being released by exonuclease before the first labeling material, and causes the both labeling materials to be apart from each other at a shortened distance, thereby to improve the quenching effect and reduce the background signal.

The exonuclease for use in the nucleic acid sequence detection method of the present invention is not particularly limited as long as it can release a nucleotide labeled with the first labeling material in the probe hybridized to the target nucleic acid. For example, λ exonuclease, T7 Gene6 exonuclease can be mentioned as the 5'exonuclease. Further, E.coli exonuclease III can be mentioned as the 3'exonuclease. These are all commercially available. Some exonuclease is unlikely to release the nucleotide at a site labeled with a labeling material or a quencher because of its steric hindrance. Even in this case, a fluorescent nucleotide analog can be used as the labeling material to be a substrate for any exonuclease, and thus can be released effectively from the probe.

If the method according to the present invention is difficult to discriminate a perfect match from a mismatch, the method can use a higher temperature to react, causing a mismatch-containing hybrid to be unstable between the target nucleic acid and the probe, thereby facilitating to discriminate the perfect match from the mismatch. For example, if the reaction at 37°C can not give a sufficient signal difference or an S/N ratio to discriminate a perfect match from a mismatch, the reaction can be executed at a temperature such as 37°C to 45°C to improve. However, a common enzyme has an optimum reaction temperature of around 37°C, and thus an elevated temperature of beyond 37°C may lower the enzyme activity to give no sufficient signal. In this case, a thermostable enzyme having a higher optimum reaction temperature is preferably used. For example, the double stranded DNA-specific 3'→5' exonuclease is isolated from Archaeoglobus fulgidus (Afu) which is a thermophilic bacteria (Patent Document 4). Such a thermostable exonuclease is utilized to widen the range of a reaction temperature to set in the present invention, providing the more suitable reaction conditions to set for various sequence targets.

Conditions for executing the present invention will be described in detail hereinafter. It should be noted that these are shown exemplarily and do not limit the present invention.

The specimen employed is a nucleic acid that can serve as a substrate for exonuclease used, and a DNA is employed as the specimen for exemplified λ exonuclease, T7 Gene6 exonuclease, exonuclease III, and 3'→5' exonuclease derived from Afu. A DNA extracted from biological samples can be used directly, but is, in many cases, provided in an insufficient amount to use for analysis. In this case, a nucleic acid extracted from a biological sample can be amplified by PCR method to give a DNA product, which is then used for sequence analysis according to the present invention. Further, the technology for reverse transcription reaction followed by amplification as DNA (such as RT-PCR) can be used to convert an RNA to the DNA, which is then amplified to give a product for analysis.

DNAs amplified by the other method than PCR such as LAMP method (Non-Patent Document 6), ICAN method (Non-Patent Document 7), Rolling Circle method (Non-Patent Document 8) may be used as the specimen for the method according to the present invention. This is because any linear-chain DNA having termini such as amplification products produced by these methods can hybridize to a probe, which can then serve as the substrate for an exonuclease. Further, even if the target nucleic acid is circular, a single-stranded circular DNA hybridizes to an oligonucleotide, which can then serve as the substrate for exonuclease III and hence can be used as a sample for the method according to the present invention. In addition to an amplified nucleic acid, the cDNA which is reverse transcribed from RNA can be used. After reverse transcription, the RNA and the cDNA give a hybrid. Therefore, the hybrid can be either denaturated to hybridize with the probe or treated with RNaseH to digest only the RNA, thereby to obtain the single-stranded cDNA, which is then hybridized with the probe to perform an exonuclease cycling assay. Thus, this allows quantitative detection and polymorphism analysis of the RNA. Further, exonuclease III, which also has RNaseH activity, can be used to perform the exonuclease cycling assay without the aid of the other enzyme having RNaseH activity.

As is in nucleotide sequence determination by Sanger method or mini-sequencing method, many enzyme reaction methods to analyze polymorphism, which uses a PCR amplification product as the specimen, is easily influenced by remaining enzyme activity, primers, deoxynucleotides, salts or pH. In many cases, the amplification product is indispensable to purify in order to remove these influences, which makes these methods extremely cumbersome to execute. The present invention is very simple and prompt to execute, because the present invention can use a PCR product or a reverse transcription product as it is unpurified to analyze as long as the reaction solution containing the product does not remarkably inhibit the exonuclease reaction and the hybridization of the probe.

In the present invention, the target nucleic acid, when it is a double-stranded DNA, is generally denaturated to the single-stranded DNA, which is then hybridized with the probe. The target nucleic acid, when it is a single-strand, needs not to be denaturated and can be hybridized with the probe DNA. The denaturation method in the present invention may be performed according to a normally employed method. For example, a thermal denaturation method and a chemical denaturation method are exemplified. Specifically, the thermal denaturation method is carried out normally by heating at a temperature of 90°C or more. More preferably, the denaturation is carried out by heating at 94°C to 98°C for about 1 to 5 min. The chemical denaturation method is carried out by actions of a denaturation agent such as urea or formaldehyde or by increasing the pH. The thermal denaturation method is preferable to employ because of the simple procedure, although the pH can be elevated by using 0.1 to 1 mol/l NaOH.

The hybridization may be performed according to a normally employed method, and the condition thereof is variable depending on length, nucleotide sequence and Tm of the probe or the denaturated target nucleic acid. In general, hybridization is carried out at pH 6.5 to 9.5, in the presence of 10 to 1000 mM sodium or potassium ion at room temperature to 70°C. However, since exonuclease activity may be impaired in the presence of excessive sodium or potassium ion, it is necessary to find out a condition which ensures a sufficient signal to noise ratio (S/N ratio) within the range of 10 to 1000 mM.

The linear double-stranded DNA as the target nucleic acid such as a PCR product may not necessarily be denaturated, if the target sequence portion of the DNA can be single-stranded by the activity of exonuclease used in the exonuclease cycling assay. For example, the both strands of the target PCR product are digested from their respective 3' termini by the catalytic activities of exonuclease III used as a 3'→5' exonuclease, allowing the PCR product to become partially single-stranded DNAs. The probe, which targets this single-stranded region as the target sequence, can be used to exempt the PCR product from a denaturation procedure. The PCR product is supplied with exonuclease III and the probe to carry out the reaction at a constant temperature, thereby to establish the exonuclease cycling reaction. Therefore, the method according to the present invention is greatly advantageous in simple and prompt procedure.

λ exonuclease is used as an example of another exonuclease. A PCR primer set, one of which is phosphorylated at the 5'-terminus, is used for amplification to get a DNA product. This DNA product is used as the target nucleic acid to digest with λ exonuclease. In the PCR product, one strand with the phosphorylated 5'-terminus is digested, while another strand with the non-phosphorylated 5'-terminus remains intact, thereby to get the single-stranded target DNA. The probe, which has a complementary sequence with the single-stranded target DNA and is phosphorylated at the 5'-terminus, can be used together with λ exonuclease to establish the exonuclease cycling assay without a denaturation procedure. The present invention can completely carry out the exonuclease cycling assay at a constant temperature, and needs no equipment for a complicated temperature control.

When λ exonuclease, T7 Gene6 exonuclease, exonuclease III are used among the exemplified exonucleases, the reaction is preferably carried out at a temperature of 25°C to 45°C. Normally, either of these enzymes is used at 37°C. However, the temperature of 37°C is not necessarily suitable in order to identify a mismatch by the present invention, because the reaction temperature is set to get a sufficiently large signal ratio between a full-match and a mismatch. In the meantime, these enzymes are used at a temperature of more than 37°C to lower their catalytic activities with the elevated temperature. Therefore, the above enzymes are not suitable for cycling assay carried out at a very high temperature such as 50°C or more. In this case, a thermostable enzyme is preferably used. As for the thermostable exonuclease, 3'→5' exonuclease derived from Afu is reported (Patent Document 4), and such enzyme is used to allow execution of preferable exonuclease cycling reaction even at a higher temperature, namely, at 50°C or more.

Enzyme concentration and probe concentration have influences on both a target nucleic acid-specific signal and a background signal. A preferable combination of enzyme concentration and probe concentration is variable depending on types of enzymes, target nucleic acid and probe sequences, and the condition should be found out to get a high S/N ratio for every case.

Fluorescence detection condition for FRET is, for example, as follows. The probe, which is modified with Dabcyl at the 5'-terminus and modified with FITC at an inner site, is digested from the 3' -terminus by the action of a 3'→5' exonuclease to emit fluorescence, which is then detected at a maximum excitation wavelength of 494 nm and at a maximum fluorescence wavelength of 518 nm using a spectrofluorometer. The method according to the present invention allows simple procedures, and therefore, facilitates construction of an automated system. Especially for construction of a high-throughput system, a microplate is conveniently used. For detection of fluorescence when a microplate is used, a fluorescence measurement apparatus suited for the microplate is used. Such apparatus includes CytoFluor Series 4000 (PerSeptive Biosystems), Wallac Arvo HTS 1420 Multilabel Counter (Perkin-Elmer Life Science), or the like, but not limited thereto. In such an apparatus used, the type of a filter attached to the apparatus limits the range of an excitation wavelength and a fluorescence wavelength to select, but is adequately selected taking account of the excitation wavelength and the fluorescence wavelength of a fluorescent dye. For example, in the case of Wallac Arvo HTS 1420 Multilabel Counter, measurements can be done preferably using an excitation filter of 485 nm and a fluorescence measurement filter of 535 nm.

A signal generated by digestion of the probe by exonuclease is amplified by cyclical reaction of the probe, and transition in the signal can be measured during progress of the reaction. The reaction time can be suitably selected to improve reproducibility and quantitative capability, and to shorten a measurement time. The reaction time is preferably from several sec to 60 min. The signal can be monitored in real-time using a real-time PCR apparatus and using a probe labeled with a fluorescent dye corresponding to the apparatuses. The real-time PCR apparatus includes PRISM 7700 Sequence Detection System (ABI), LightCyler (Roche Diagnostics), and Smart Cycler II System (Takara Bio Inc.). Any of these apparatuses is able to control strictly a reaction temperature of 4°C to 100°C, is applicable for fluorescence measurement at various wavelengths, and can be used preferably in the present embodiment.

Further, the present invention can identify one or a plurality of mutations which are present in a region of several continuous bases, in addition to polymorphism at a specific site. For example, the K-ras gene is known to work as a carcinogenic gene when a mutation occurs in the 12th codon (GGT) to be accompanied with an amino acid substitution, particularly with the substitution of glycine for valine, asparatic acid or arginine. The mutation in K-ras gene is detected in about 40% of intestinal cancers, about 20% of lung cancers, 80 to 90% of pancreas cancers, and is utilized also for assay of clinical specimens. The thymine, which is a third base in the 12th codon, is substituted for any other base to be accompanied with no amino acid substitution, while the first base or the second base is substituted for any other base to be accompanied with an amino acid substitution. Therefore, in order to utilize the technology for examining site-specific single nucleotide polymorphism, it is indispensable to prepare the totally seven sets of probes corresponding to the substitution of the first base and the second base, in addition to the wild-type sequence. However, according to the present invention, any base substitution in a region of continuous 3 or more bases brings a remarkable reduction in fluorescence intensity, allowing discrimination from a specific sequence. The method according to the present invention is effective for detecting not only single nucleotide polymorphism but also a mutation in a cancer-related gene.

### Example

Hereinafter, the present invention will be described in detail referring to Examples. However, the present invention is not limited thereto.

### Example 1

### (1) Preparation of target nucleic acid

A part of K-ras gene from human genome DNA was amplified by PCR to give a product, which was then used as a target nucleic acid. The upstream and downstream primers had their respective sequences as shown below. All the oligomer DNAs shown below were synthesized by the Japan Bio Services Co., Ltd.

SEQ ID NO: 1 : GAGAGGCCTGCTGAAAATG
SEQ ID NO: 2: CCTCTATTGTTGGATCATATTC
As for PCR conditions, 50 µl of reaction solution containing 1. 25 U of Ex Taq DNA Polymerase (Takara Bio Inc.), 1X Ex Taq buffer solution (Takara Bio Inc.), 200 µM of each dNTP, 200 nM of each primer, 10 to 100 ng of human genome DNA was prepared, and the cycling reaction of 94°C/20 sec, 55°C/20 sec, 72°C/20 sec was carried out 40 times. The cycling reaction was carried out similarly using a reaction solution which was free from human genome DNA to give a negative control.

### (2) Probe DNA

A probe which was labeled with Dabcyl at the 5'-terminus and with FITC at an inner site of the probe had the same base sequence as a part of human K-ras gene as follows: wherein "Z" in the sequence denotes the FITC-labeled deoxythymidine (FITC-dT).

SEQ ID NO: 3: 5'-GCTCCAACZACCACAAGTTTATATTCAGTC

### (3) Exonuclease cycling assay

The amplification product of K-ras gene of above-mentioned (1) was quantitatively determined by PicoGreen dsDNA Quantitation Kit (Molecular Probe), and then diluted with the negative control by two-fold serial dilution. The series of the two-fold dilution and the negative control were assayed using a probe having a sequence shown by SEQ ID NO: 3 and labeled with Dabcyl at the 5'-terminus by exonuclease cycling assay. Namely, 10 µl of reaction solution containing 2X exonuclease III Buffer (Takara Bio Inc.), 75mM NaCl, 750 nM probe DNA, 2 µl of specimen DNA (PCR product) was prepared, heated at 95°C for 2 min using Mastercycler ep (Eppendorf), and then heated at 37°C for 5 sec. The resultant solution was immediately supplied with 10 µl of 1.35 U/µl exonuclease III, and kept at 37°C for further 30 min, and heated at 70°C for 10 min to deactivate the enzyme, and then supplied with 30 µl of TE buffer solution (10 mM Tris-HCl, 1 mM EDTA, pH 8.0) to make a total volume, which was then transferred in a 96-well microplate (Corning) for fluorescence measurement. The fluorescence was measured at an excitation wavelength of 485 nm and at a fluorescence wavelength of 535 nm using the plate reader Wallac Arvo HTS 1420 Multilabel Counter (Perkin-Elmer Life Science).

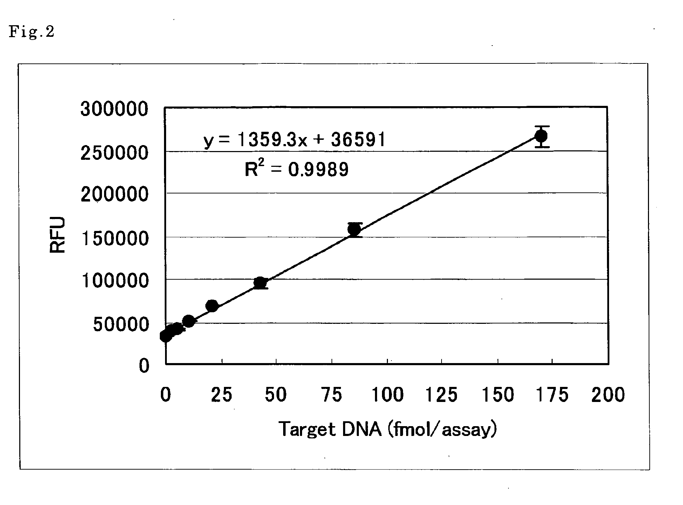

As a result, as shown in FIG. 2, the standard curve obtained was excellent in linearity, and the minimum detection sensitivity was 3 fmol/assay or less.

### Example 2

### (1) Assay with or without denaturation step

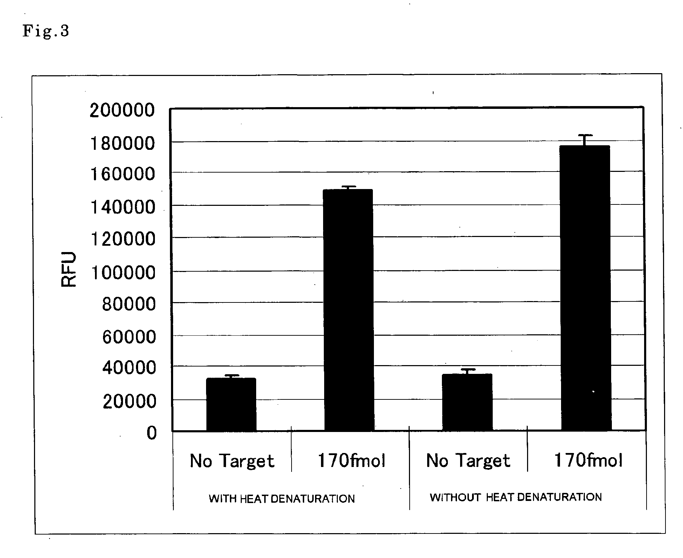

The method shown in above Example 1 (3) includes a step for heat denaturating the double-stranded target DNA prior to addition of exonuclease. In order to verify whether or not this step is essential, 170 fmol K-ras PCR product prepared in above Example 1 (1) and the negative control were subjected to the method of above Example 1 (3) with or without the heat denaturation step included, and then compared to verify. The method without the heat denaturation step included was carried out in such a process that 20 µl of the reaction solution containing 1X Exonuclease III Buffer (Takara Bio Inc.), 37.5 mM NaCl, 375 nM probe, 2 µl of specimen DNA (170 fmol PCR reaction solution), and 0.675 U/µl of exonuclease III was prepared, maintained at 37°C for 30 min, heated at 70°C for 10 min to deactivate the enzyme, and the fluorescence was measured at an excitation wavelength of 485 nm and at a fluorescence wavelength of 535 nm using the plate reader Wallac Arvo HTS 1420 Multilabel Counter (Perkin-Elmer Life Science). As a result, as shown in FIG. 3, with regard to both a signal of the PCR reaction solution without the target nucleic acid contained and a signal of the PCR reaction solution with 170 fmol target sequence contained, the method without the heat denaturation step included gave values which were nearly identical to those of the method with the thermal denaturation step included. From this, it has been revealed that the heat denaturation step is not essential.

### Example 3

### (1) Synthesized target nucleic acid

In order to verify the ability of exonuclease cycling assay to identify substitution of single base, 13 kinds of chemically synthesized DNAs were used as the target nucleic acid. The nucleotide sequences of the chemically synthesized target DNAs are as follows:

SEQ ID NO: 4: 5'-GAAAATGACTGAATATAAACTTGTGGTAGTTGGAGCTTGT
SEQ ID NO: 5: 5'-GAAAATGACTGAATATAAACTTGTTGTAGTTGGAGCTTGT
SEQ ID NO: 6: 5'-GAAAATGACTGAATATAAACTTGTGTTAGTTGGAGCTTGT
SEQ ID NO: 7: 5'-GAAAATGACTGAATATAAACTTGTGGCAGTTGGAGCTTGT
SEQ ID NO: 8: 5'-GAAAATGACTGAATATAAACTTGTGGTGGTTGGAGCTTGT
SEQ ID NO: 9: 5'-GAAAATGACTGAATATAAACTTGTGGTATTTGGAGCTTGT

SEQ ID NO: 10: 5'-GAAAATGACTGAATATAAACTTGTGGTAGCTGGAGC

TTGT

SEQ ID NO: 11: 5'-GAAAATGACTGAATATAAACTTGTGGTAGTCGGAGC
TTGT

SEQ ID NO: 12: 5′-GAAAATGACTGAATATAAACTTGTGGTAGTTTGAGC

TTGT

SEQ ID NO: 13: 5′-GAAAATGACTGAATATAAACTTGTGGTAGTTGTAGC

TTGT

SEQ ID NO: 14: 5'-GAAAATGACTGAATATAAACTTGTGGTAGTTGGCGC
TTGT

SEQ ID NO: 15: 5'-GAAAATGACTGAATATAAACTTGTGGTAGTTGGATC
TTGT

SEQ ID NO: 16: 5′-GAAAATGACTGAATATAAACTTGTGGTAGTTGGAGA
TTGT.

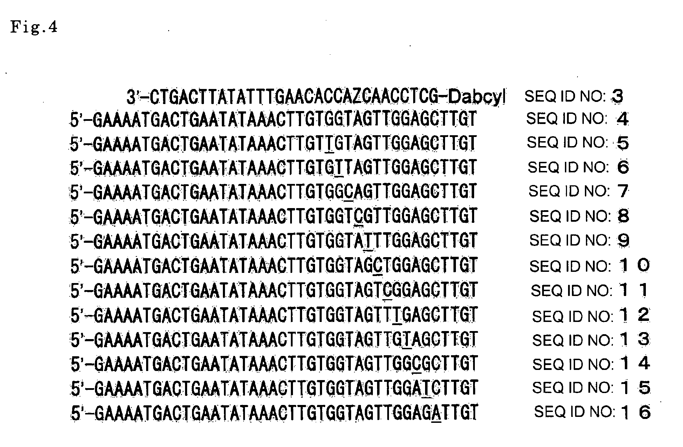

Here, SEQ ID NO: 4 is a wild-type sequence of human K-ras gene and includes a sequence completely complementary to the nucleotide sequence of the probe DNA shown by SEQ ID NO: 3. Meanwhile, SEQ ID NO: 5 through SEQ ID NO: 16 are each the nucleotide sequences shown by SEQ ID NO: 4 with any one base substituted for the other base, and hybridized to the probe DNA shown by SEQ ID NO: 3 to form double-stranded DNAs with their respective one base mismatche included. Their respective positions of mismatches are shown in FIG. 4. The bases designated by the underlines in FIG. 4 form their respective mismatches by hybridizing to the probe DNA shown by SEQ ID NO: 3.

### (2) Detection of one base mismatch using synthesized target nucleic acid

In order to verify the ability of the present invention to identify one base mismatch using the synthesized target nucleic acid, exonuclease cycling assay was carried out under the following conditions. Namely, 20 µl of reaction solution containing 1X Exonuclease III Buffer (Takara Bio Inc.), 37.5 mM NaCl, 375 nM probe DNA, 0.675 U/µl exonuclease III (Takara Bio Inc.), 100 fmol synthesized target DNA, and 20 µl of reaction solution containing the same except that nuclease-free water was contained instead of the synthesized target DNA were prepared in PCR reaction tubes, incubated immediately at 37°C or 41°C for 30 min using Mastercycler ep (Eppendorf), heated at 70°C for 10 min to deactivate the enzyme, and supplied with 30 µl of TE buffer solution to make a total volume, which was then transferred in 96-well microplate (Corning) for fluorescence measurement. The fluorescence was measured at an excitation wavelength of 485 nm and at a fluorescence wavelength of 535 nm using plate reader Wallac Arvo HTS 1420 Multilabel Counter (Perkin-Elmer Life Science). The relative fluorescence unit (RFU) for the sample with the synthesized target DNA contained was subtracted from that for the sample with nuclease-free water contained to give a net value (ΔRFU).

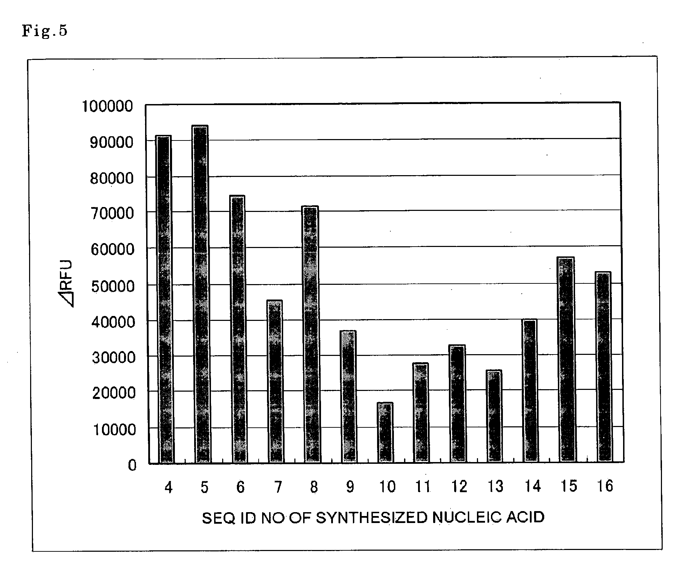

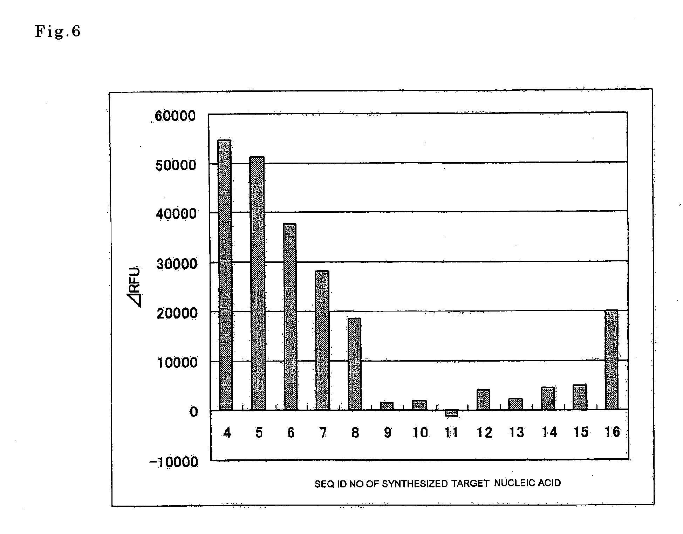

As a result, it was observed that the reaction at 37°C reduced more or less the signals of the target nucleic acids shown by SEQ ID NO: 7 and SEQ ID NOS: 9 through 16, compared with that of the perfect match target nucleic acid shown by SEQ ID NO: 4 (FIG. 5). Next, it was observed that the reaction at 41°C reduced remarkably the signals of the target nucleic acids shown by SEQ ID NOS: 9 through 15, and that the signals had a nearly same level as the background signal, that is, a signal without the target nucleic acid contained. It was revealed that these target nucleic acids which form a mismatch with the probe could be discriminated from the other target nucleic acids which had a completely complementary sequence with the probe (FIG. 6). This fact indicates that the present invention can detect a gene mutation not only in one specific site but also in a region of several bases around the site.

### Example 4

### (1) Synthesized target nucleic acid

In order to verify the ability of exonuclease cycling assay to identify a sequence with one or more bases insertion or deletion, four types of chemically synthesized DNAs were used as the target nucleic acid. The chemically synthesized target DNAs had their respective base sequences as follows:

SEQ ID NO: 17: 5'-GAAAATGACTGAATATAAACTTGTGGTAGTGGAGCTTGT
SEQ ID NO: 18: 5'-GAAAATGACTGAATATAAACTTGTGGTAGGAGCTTGT

SEQ ID NO: 19: 5′-GAAAATGACTGAATATAAACTTGTGGTAGTGTGGAGCTT
GT

SEQ ID NO: 20: 5′-GAAAATGACTGAATATAAACTTGTGGTAGTGAATGGAGC
TTGT.

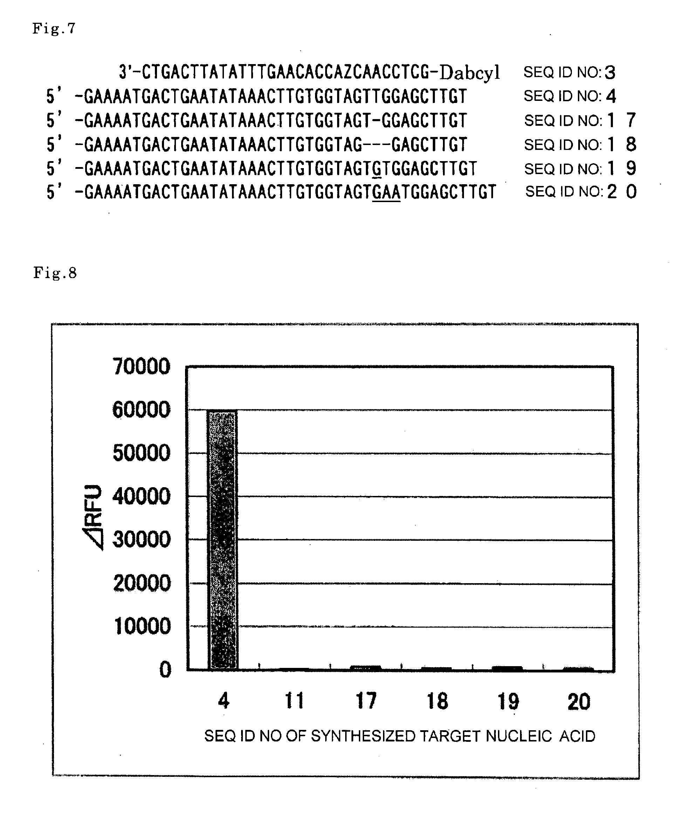

In FIG. 7, SEQ ID NO: 4 is a wild-type sequence of human K-ras gene and includes a sequence completely complementary to the nucleotide sequence of the probe DNA shown by SEQ ID NO: 3. Meanwhile, SEQ ID NO: 17 through SEQ ID NO: 20 are each the nucleotide sequences shown by SEQ ID NO: 4 with one or three bases insertion or deletion, and hybridized to the probe DNA shown by SEQ ID NO: 3 to form double-stranded DNAs with a loop structure in either strand. The spaces designated by the hyphen(s) in FIG. 7 show nucleotide deletions, and the bases designated by the underlines show inserted nucleotides.

### (2) Detection of inserted and deleted sequences using synthesized target nucleic acids

In order to verify the ability of the present invention to identify inserted and deleted sequences using synthesized target nucleic acids, exonuclease cycling assay was carried out under the following conditions. Namely, 20 µl of reaction solution containing 1X Exonuclease III Buffer (Takara Bio Inc.), 37. 5 mM NaCl, 375 nM probe DNA, 0.675 U/µl exonuclease III (Takara Bio Inc.), 300 fmol synthesized target DNA, and 20 µl of reaction solution containing the same except that nuclease-free water was contained instead of the synthesized target DNA were prepared in PCR plates for fluorescence measurement, incubated immediately at 41°C for 30 min using Mastercycler ep (Eppendorf), heated at 70°C for 10 min to deactivate the enzyme, and supplied with 30 µl of TE buffer solution to make a total volume, which was then transferred in 96-well microplate (Corning) for fluorescence measurement. The fluorescence was measured at an excitation wavelength of 485 nm and at a fluorescence wavelength of 535 nm using plate reader Wallac Arvo HTS 1420 Multilabel Counter (Perkin-Elmer Life Science). The relative fluorescence unit (RFU) for the sample with the synthesized target DNA contained was subtracted from that for the sample with nuclease-free water contained to give a net value (ΔRFU).

As a result, it was observed that the synthesized target DNA shown by SEQ ID NO: 11 having one base substitution and the synthesized target DNAs shown by SEQ ID NOS: 17 through 20 including insertion and deletion gave remarkably lower fluorescence intensities than the synthesized target nucleic acid shown by SEQ ID NO: 4 (FIG. 8). From this, it has been revealed that the method according to the present invention can discriminate a target nucleic acid having a specific sequence from the other target nucleic acids having insertion or deletion sequence of one or more bases.

### Example 5

### (1) PCR of human aldehyde dehydrogenase 2 gene

In order to demonstrate that the present invention can analyze polymorphism in a target nucleic acid which is not only chemically synthesized but also derived from an actual living specimen, there was constructed a polymorphism detection system of human aldehyde dehydrogenase 2 (ALDH2) gene. Single nucleotide polymorphism of normal type "G" and mutant type "A" is present at exon 12 of ALDH2 gene. In order to identify this polymorphism by exonuclease cycling assay, a region containing this polymorphism site was amplified by PCR from the sample DNAs of which the genotype of ALDH2 gene is already known. Sequences of the primers used in PCR are as follows:

SEQ ID NO: 21: GTGGCCGGGAGTTGGGCGAG
SEQ ID NO: 22: GCCCCCAACAGACCCCAATC.

As for PCR conditions, 25 µl of reaction solution containing 1X Ex Taq Buffer (Takara Bio Inc.), 200 µM each of dNTP, 200 nM each of primer, 0.625 U of Ex Taq DNA polymerase (Takara Bio Inc.), 10 to 50 ng of human genome DNA was prepared, then subjected to PCR as follows: denaturated at 94°C/2 min as an initial denaturation, followed by cycling reaction consisting of 40 cycles of 94°C/20 sec, 63°C/20 sec, and 72°C/20 sec, with final extension at 72°C/2 min. Success in the amplification was confirmed by 2% agarose gel electrophoresis and visualization by ethidium bromide staining.

### (2) Specific detection of wild-type ALDH2 gene sequence by exonuclease cycling assay

20 µl of reaction solution containing 1X Exonuclease III Buffer (Takara Bio Inc.), 40 mM NaCl, 150 nM probe DNA, 0.4 U/µl exonuclease III (Takara Bio Inc.), and 5 µl of above-mentioned PCR reaction solution or nuclease-free water was prepared in a PCR reaction tube, and incubated immediately at 37°C for 30 min using Mastercycler ep (Eppendorf), and heated at 70°C for 10 min to deactivate the enzyme, and supplied with 30 µl of TE buffer solution to make a total volume, which was transferred in 96-well microplate (Corning) for fluorescence measurement. The fluorescence was measured at excitation wavelength of 485 nm and at a fluorescence wavelength of 535 nm by plate reader Wallac Arvo HTS 1420 Multilabel Counter (Perkin-Elmer Life Science). The probe DNA used here comprised a sequence that completely matched with the target nucleic acid having normal type "G." The sequence thereof was as follows:

SEQ ID NO: 23: TTTCACTTCAGZGTATGCCTGCAGCC.

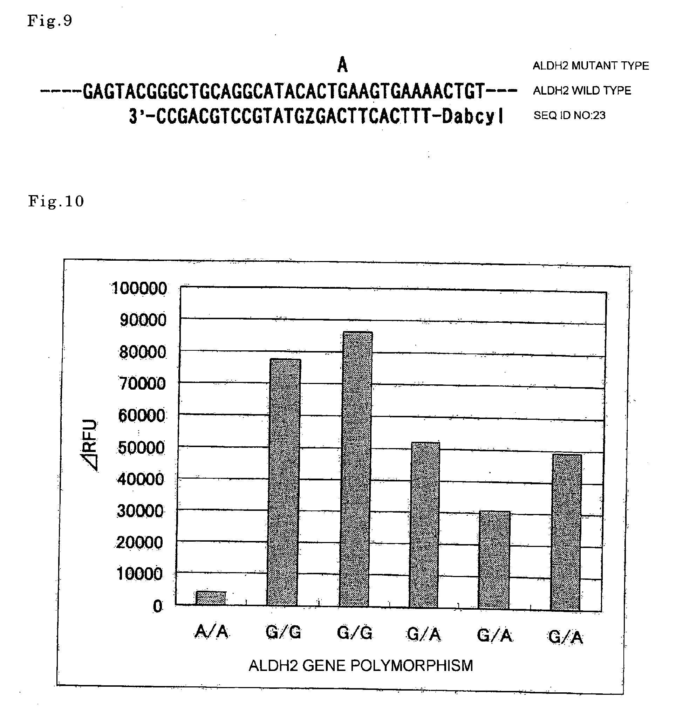

Here, Z denotes a dT labeled with FITC, and the 5'-terminus of the probe was labeled with Dabcyl that is a dark quencher (FIG. 9). As a result, A/A type showed a remarkably lower RFU-value than G/G type and G/A type (FIG. 10) . From this, it has been demonstrated that the method according to the present invention can identify definitely whether one base is substituted in a living specimen or not.

### Example 6

### (1) Probe introduced with artificial mismatch

Of mismatched base pairs, G/G and G/T base pairs have comparatively strong hydrogen bonds, and hence are more difficult to discriminate distinctly from completely matched base pairs to detect using an ordinary hybridization method than the other mismatched base pairs. The present invention also has the similar tendency, but can avoid this problem by intentionally replacing partly a probe sequence and forming a mismatch with regard to the target sequence. In order to exhibit one example of this, there were designed two types of probes for discriminating "A" included in the mutant type ALDH2 from "G" included in the wild-type to detect as shown below.

SEQ ID NO: 24: CACTTTAGTGZATGCCTGCAGCCCGTA
SEQ ID NO: 25: CACTTTAGTGZATGTCTGCAGCCCGTA

Here, Z denotes a dT labeled with FITC, and the 5'-terminus of the probe was labeled with Dabcyl that is a dark quencher.

Moreover, oligomer DNAs comprising each the wild-type sequence and the mutant type sequence of ALDH2 as shown below were synthesized as target nucleic acid.

SEQ ID NO: 26: GAGTACGGGCTGCAGGCATACACTGAAGTGAAAACTGT
SEQ ID NO: 27: GAGTACGGGCTGCAGGCATACACTAAAGTGAAAACTGT

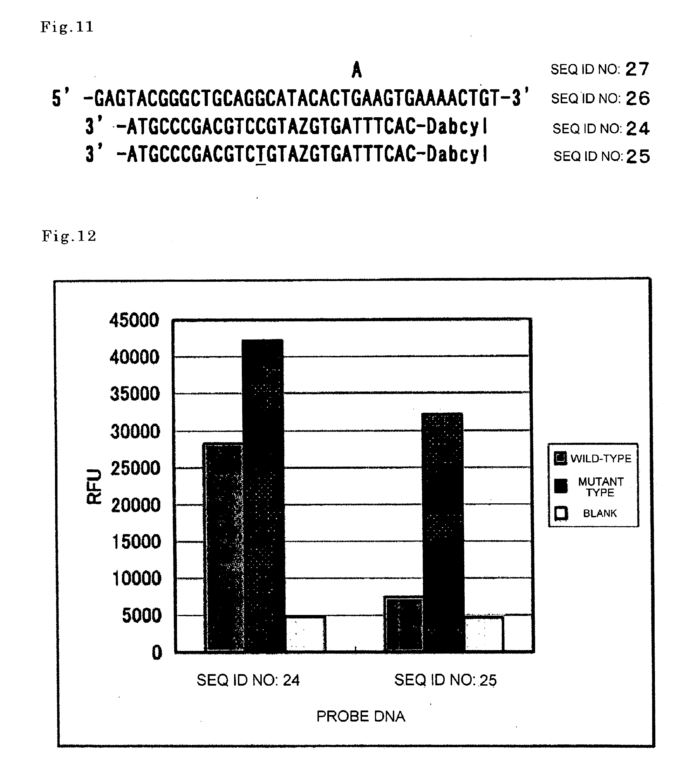

A relationship between these probes and the target DNA is shown in FIG. 11. The probe comprising SEQ ID NO: 24 is completely complementary in nucleotide sequence to the mutant type target DNA comprising SEQ ID NO: 27, while it forms a G/T mismatch base pair with the wild-type target DNA comprising SEQ ID NO: 26 at the polymorphism site. Meanwhile, the probe comprising SEQ ID NO: 25 has a substitution of from C to T (underlined) at the fourth base counted from the site labeled with FITC towards 3' side in SEQ ID NO: 24. Thus the probe was designed to form a mismatched base pair at the substituted site with both wild-type and mutant type target DNAs.

### (2) Improvement of capability of polymorphism identification by introducing artificial mismatch to a probe

Using these DNAs, 20 µl of reaction solution containing 1X Exonuclease III Buffer (Takara Bio Inc.), 40 mM NaCl, 250 nM probe DNA, 0.05 U/µl exonuclease III (Takara Bio Inc.), 100 fmol synthesized target DNA was prepared in 96-well PCR plate for fluorescence measurement, incubated immediately at 37°C for 30 min using Mastercycler ep (Eppendorf), heated at 70°C for 5 min to deactivate the enzyme. The fluorescence was measured at an excitation wavelength of 485 nm and at a fluorescence wavelength of 535 nm by plate reader Wallac Arvo HTS 1420 Multilabel Counter (Perkin-Elmer Life Science). Moreover, as a blank, reaction solution without the target DNA contained was likewise treated to measure fluorescence.

Results obtained are shown in FIG. 12. The probe, which completely matched with the wild-type sequence, gave the RFU values too much elevated from the blank value with regard to both of the wild-type target DNA and the mutant-type target DNA to discriminate them by a difference in signal value. However, the probe introduced with the artificial mismatch gave a remarkable difference in RFU values between the wild-type target DNA and mutant type target DNA, and thus could improve the specificity to identify single nucleotide polymorphism.

### Example 7

### (1) Determination of ALDH2 genotype in living specimen

The optimized probes were designed to detect specifically the wild type and the mutant type of ALDH2 gene, and used to optimize measurement conditions for exonuclease cycling assay. A sample derived from human was attempted to identify the genotype of the ALDH2 gene.

1 to 50 ng of human genome DNA from 23 individuals and nuclease-free water in lieu of the DNA as the no template control (NTC) were used to amplify the region including a polymorphism site of ALDH2 gene by way of the same method as in Example 5, and then 5 µl of the PCR product was assayed using the wild-type detection system and the mutant type detection system of ALDH2 gene, respectively. Namely, 20 µl of reaction solution containing 1X Exonuclease III Buffer (Takara Bio Inc.), 40 mM NaCl, 150 nM probe DNA, 5 µl of the PCR product, exonuclease III (Takara Bio Inc.) of 0.2 U/µl (wild-type detection system) or 0.0375 U/µl (mutant type detection system) on thermal cycler at 4 °C was prepared, incubated at 37°C/30 min and then at 70°C/5 min. The fluorescence was measured at an excitation wavelength of 485 nm and at a fluorescence wavelength of 535 nm by plate reader Wallac Arvo HTS 1420 Multilabel Counter (Perkin-Elmer Life Science). Sequences of the probes used for specific detection of the wild-type and the mutant type on this occasion are shown in SEQ ID NOS: 23 and 25.

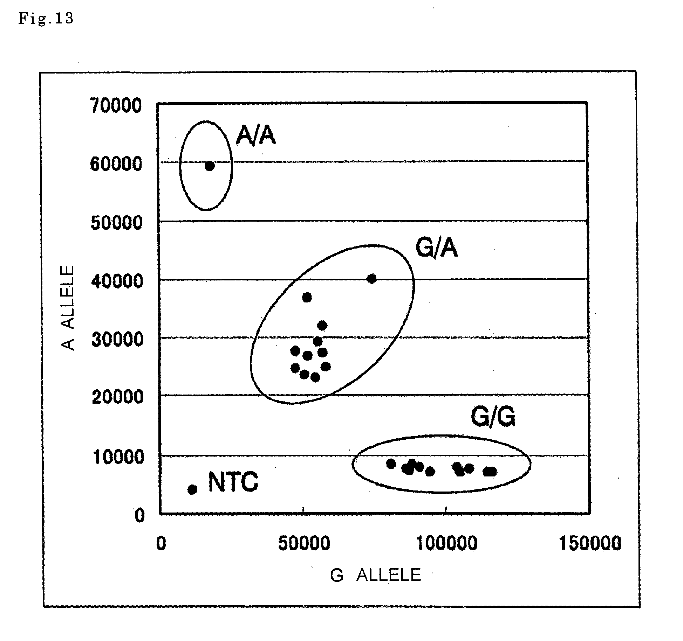

Distribution of fluorescence signals of each specimen is shown in FIG. 13, where the horizontal axis (G allele) represents the RFU-value by the wild-type detection system and the vertical axis (A allele) represents the RFU-value by the mutation detection system. Distribution of fluorescence signals was clearly divided into three groups, and the groups each coincided completely with three specimen groups of G/G homo, G/A hetero, A/A homo, the genotypes of which were determined by the Sanger method. From this, it has been demonstrated that the present invention is extremely useful for determining genotypes of actual living specimens.

### Example 8

### (1) Quantitative determination of allele content in mixed specimen

The genome DNA which had an ALDH2 genotype of G/G homo and the genome DNA which had an ALDH2 genotype of A/A homo were mixed to prepare samples which had each a G/G homo genome DNA content of 100%, 75%, 50%, 25%, or 0%. 10 ng of these mixed samples were each subjected to PCR by way of the same method as in Example 5. 5 µl of these PCR products were each assayed in duplicate using the wild-type detection system and the mutant type detection system of ALDH2 gene by way of the same method as in Example 7.

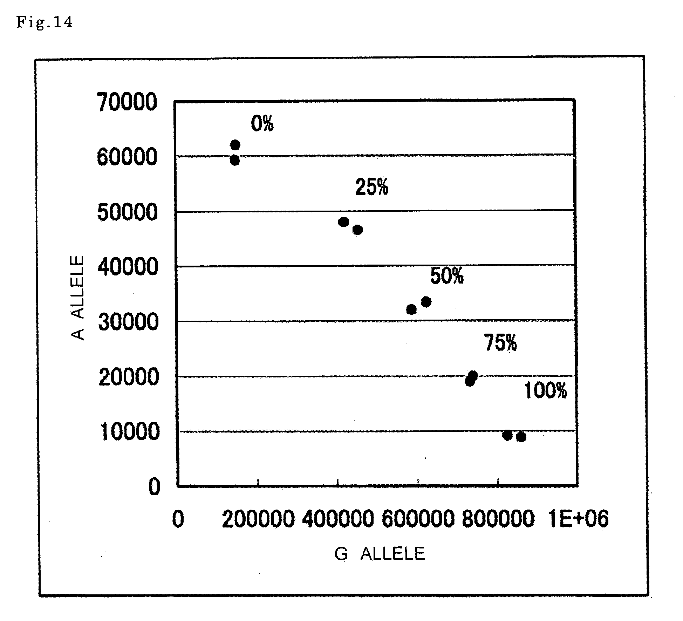

As a result, the samples having their respective mix ratios showed each their independently distributed signals. It has been revealed that the content of each allele can be quantitatively determined (FIG. 14).

### Brief Description of the Drawings

FIG. 1 is a drawing showing principle of exonuclease cycling assay which allows homogeneous detection of gene polymorphism or gene mutation.
FIG. 2 is a drawing showing a standard curve in homogeneous detection system of exonuclease cycling assay. (Example 1)
FIG. 3 is a drawing showing results of measurement with and without a step for heat denaturating DNA prior to addition of exonuclease. (Example 2)
FIG. 4 is a drawing showing sequences of a probe and synthesized target nucleic acids. (Example 3)
FIG. 5 is a drawing showing results of mismatch detection attempted by homogeneous detection system of exonuclease cycling assay performed at a reaction temperature of 37°C. (Example 3)
FIG. 6 is a drawing showing results of mismatch detection attempted by homogeneous detection system of exonuclease cycling assay performed at a reaction temperature of 41°C. (Example 3)
FIG. 7 is a drawing showing sequences of a probe and synthesized target nucleic acids. (Example 4)
FIG. 8 is a drawing showing results of insertion and deletion sequence detection attempted by homogeneous detection system of exonuclease cycling assay. (Example 4)
FIG. 9 is a drawing showing sequences of a region including single nucleotide polymorphism of ALDH2 gene and sequence of the wild-type ALDH2 gene-specific probe DNA. (Example 5)
FIG. 10 is a drawing showing results of polymorphism detection of ALDH2 gene by homogeneous detection system of exonuclease cycling assay. (Example 5)
FIG. 11 is a drawing showing sequences of probes and synthesized target nucleic acids. (Example 6)
FIG. 12 is a drawing showing results of an attempt to improve specificity of one base substitution detection by using a probe introduced with artificial mismatch in exonuclease cycling assay. (Example 6)
FIG. 13 is a drawing showing results of identification of genotype of ALDH2 from living specimen by exonuclease cycling assay. (Example 7)
FIG. 14 is a drawing showing results of exonuclease cycling assay of a specimen prepared by mixing two human genome DNAs different in genotype of ALDH2 at different mix ratios. (Example 8)

## Claims

1. A method of homogeneous detection system for discriminating a target nucleic acid having a specific sequence from the other nucleic acid which has the sequence of the target nucleic acid comprising at least one or more bases substitution, insertion or deletion, thereby to detect quantitatively, the method comprising the steps of:
I) hybridizing an oligonucleotide probe containing a labeling material to a target DNA;
II) digesting the hybridized oligonucleotide probe by a double-stranded DNA-specific 5'→3' or 3'→5' exonuclease; and
III) detecting a signal emitted by at least one labeling material released from the digested oligonucleotide probe.

2. The method according to Claim 1, wherein the target nucleic acid, which has a specific site to form at least one or more non-complementary base pairs with the probe or has the sequence of the target nucleic acid with one or more bases substituted, inserted, or deleted to form a loop structure, does not hybridize with the probe or hybridizes, but leaves the probe before digestion by a 5'→3' exonuclease or a 3'→5' exonuclease, so that the probe is exempted from digestion to release no origonucleotide containing the labeling material, resulting in no significant increase of the signal.

3. The method according to Claim 1 or 2, wherein the exonuclease is a 5'→3' exonuclease, and the oligonucleotide probe is designed to form the at least one or more non-complementary base pairs or the loop structure with regard to the target nucleic acid so that the base pairs or the loop structure may be positioned between the 3'-terminal of the probe and a site labeled with the labeling material.

4. The method according to Claim 1 or 2, wherein the exonuclease is a 3'→5' exonuclease, and the oligonucleotide probe is designed to form the at least one or more non-complementary base pairs or the loop structure with regard to the target nucleic acid so that the base pairs or the loop structure may be positioned between the 5'-terminal of the probe and a site labeled with the labeling material.

5. An oligonucleotide probe for use in the method according to Claim 3, wherein the labeling material is a fluorescent dye, toward the 3'-side from which the probe is labeled with a quenching material which has an effect to attenuate efficiently fluorescence emitted by the fluorescent dye.

6. An oligonucleotide probe for use in the method according to Claim 3, wherein the labeling material is a quenching material, toward the 3' -side from which the probe is labeled with a fluorescent dye the fluorescence of which can be attenuated efficiently by the quenching material.

7. An oligonucleotide probe for use in the method according to Claim 4, wherein the labeling material is a fluorescent dye, toward the 5'-side from which the probe is labeled with a quenching material which has an effect to attenuate efficiently the fluorescence emitted by the fluorescent dye.

8. An oligonucleotide probe for use in the method according to Claim 4, wherein the labeling material is a quenching material, toward the 5' -side from which the probe is labeled with a fluorescent dye the fluorescence of which can be attenuated efficiently by the quenching material.

9. An oligonucleotide probe for use in the method according to Claim 3 or 4, wherein the labeling material is a fluorescent nucleotide analog which can form a Watson-Crick base pair with any of guanine, adenine, thymine, and cytosine in the target DNA.

10. The oligonucleotide probe according to Claim 9, wherein the fluorescent nucleotide analog is 2-aminopurine.

11. The method according to Claim 3, wherein the 5'→3' exonuclease is λ exonuclease or T7 Gene6 exonuclease.

12. The method according to Claim 4, wherein the 3'→5' exonuclease is exonuclease III.

13. The method according to any one of Claims 1 through 4, wherein the exonuclease is a thermostable enzyme.

14. The method according to Claim 13, wherein the thermostable enzyme is derived from Archaeoglobus fulgidus.

15. The method according to Claim 1 or 2, wherein a cDNA synthesized from RNA by reverse transcription reaction, or a product amplified by polymerase chain reaction (PCR) or reverse transcription-PCR (RT-PCR) is not purified to be used as a test specimen.

16. The method according to any one of Claims 1 to 4 and 11 to 15, comprising using the oligonucleotide probe according to any one of Claims 5 to 10, wherein the oligonucleotide probe has been intentionally introduced with the one or more bases substitution, insertion or deletion to form a non-complementary base pair or a loop structure with regard to the target nucleic acid, thereby to get the improved ability to discriminate the target nucleic acid from a nucleic acid which has the sequence of the target nucleic acid comprising at least one or more bases of substitution, insertion or deletion.

17. A measurement reagent or a kit for use in the method according to any one of Claims 1 through 16.
